# EUROPÄISCHE PATENTANMELDUNG

(11) **EP 1 552 790 A1**
(43) Veröffentlichungstag der Anmeldung: **13.07.2005**
(21) Anmeldenummer: 04000115.8
(22) Anmeldetag: 07.01.2004
(51) Int. Cl.: A61B 10/00

(54) **Vorrichtung an der Mundschleimhaut zur Analyse von biochemischen Prozessen oder Molekülen**

(71) Anmelder: Schwertner, Heiko, Dr., 19055 Schwerin (DE)
(72) Erfinder: Schwertner, Heiko, Dr., 19055 Schwerin (DE)

(57) **Zusammenfassung**

Ziel ist es, die bestehenden Nachteile der zur zeit vorherrschenden Verfahren und Vorrichtungen für Probennahme und Applikation in der Mundschleimhaut oder Darmmucosa zu vermeiden. Ferner ein Testverfahren zur teilweisen Testsystem von Reaktionskaskaden aufzubauen, mit dem es möglich ist in vivo Reaktion in vitro Messen zu können.

Das Problem wird durch einen Trägerfläche (1) mit einer Kopplungsfläche (3) auf welcher kovalent Substanzen oder Gemische gebunden sind, die durch eine poröse Gaze oder anderer Befestigung am Trägerfläche (1), und anschließenden Verfahren welche durch die Kontaktierung an der Mundschleimhaut gelöst. Die Gesamtheit aus Aufbauweise des Trägerfläche (1) mit Kopplungsfläche (3) und Gaze und Art der Ausgestaltung der Substanzen welche an der Kopplungsfläche (3) gebunden sind ist der Vorrichtung, welcher Teile von Reaktionskaskaden von Erkrankungsgeschehen in vivo nachbilden kann, so dass eine ex vivo also in vitro Bestimmung von Testparametern mit dem Stand der Technik entsprechenden analytischen Methoden möglich ist.

## Beschreibung

### Hintergrund der Erfindung

Ethische und moralische Grundsätze verbieten es im Normalfall biochemische Tests direkt am Menschen durchzuführen. Deshalb werden, wenn dies notwendig wird z.B. im Erkrankungsfall oder zu Testzwecken, Proben genommen z.B. Blut, Gewebe, Zellen etc. oder es werden Substanzen wie Medikamente, Antibiotika etc. mit Hilfe von Applikatoren eingebracht bzw. appliziert.
Sinnvoll wäre es, zu mindest einen Teil der Reaktionen des Körpers direkt messen zu können. Damit wäre es möglich auf einen großen Teil der Probennahme oder Applikationen verzichten zu können, welche meist mit großen Aufwand und Schmerzen für den Patienten verbunden ist, wie z.B. bei der lebend Gewebeprobennahme in der Nasenschleimhaut, Mundschleimhaut oder bei der Darmmucosa.
Da es für verschiedene Testverfahren notwendig ist vor den eigentlichen Test oder Testverfahren Moleküle oder Molekülgemische zu applizieren, wäre es sinnvoll und notwendig ein universelles Testverfahren zur Verfügung zu haben, dass Reaktionen an äußeren Gewebeschichten, wie der Mundschleimhaut direkt oder indirekt messen kann und/oder zu gleich für Testverfahren notwendige Moleküle oder Molekülgemische zu applizieren in der Lage ist. Die vorliegende Erfindung stellt hierzu einen neuen praktikablen Ansatz dar.

### Stand der Technik

Es sind verschiedene Verfahren und Vorrichtungen bekannt, wie Probennehmer z.B. US 4,800,896 vom 31.01.1989 oder Applikatoren wie z.B. US 4,887,611 vom 19.12.1989 mit denen es möglich ist entweder Proben zu entnehmen oder Substanzen zu applizieren. Ferner sind in der wissenschaftlichen Literatur verschiedene Probennehmer oder Applikatoren beschreiben wie z.B. bei F. Marcucci und L. Sensi in Clinical and Experimental Allergy, 1989 Vol.19, Seite 157 - 162 mit dem Titel "A new method for IgE detection in nasal mucosa".
In der Patentschrift DE69112610T2 mit dem Titel "Sammlung von Analyten aus der Mundschleimhaut für Immunoassays", wird ein beschrieben welches mittels passiven Aufnehmens von Körperflüssigkeiten Proben entnimmt. In der Patentschrift DE69423342T2 mit dem Titel "Transmukosale Verabreichung von makromolekularen Medikamenten", wird ein Verfahren beschrieben, welches in der Lage ist größere Moleküle, durch Auflegen eines Flieses, in die Mundschleimhaut diffundieren zu lassen. Vergleichbares ist, dem Prinzip nach, auch in der Patentschrift DE3618553C2 mit dem Titel "Medizinischer Klebestreifen für die Mundschleimhaut" zu entnehmen. In der Patentschrift DE19622503C2 mit dem Titel "Verfahren zum Nachweis von Analyten auf einer Oberfläche" werden die Analyten von der Mundschleimhaut gewischt und anschließende in situ mit einem lateral Flow Test nachgewiesen.

### Vorteile der Erfindung

Die Erfindung ersetzt komplett Probennehmer und Applikatoren in einer einzigen Einheit, wobei ein oder mehrere Parameter (Analyt) nachgewiesen werden kann.
In der erfindungsgemäßen Vorrichtung und Verfahren wird ein Teil einer komplexen biochemischen Reaktion des Körper derart vorweggenommen, dass eine Antwort des Körper auf einen äußeren Reitz in vivo extern also in vitro bestimmt bzw. gemessen werden kann. Die geschied durch auf der Oberfläche der Vorrichtung unmittelbar oder mittelbar über Schichten oder Membranen, gebundene Antigen, Antikörper, Enzyme , DNA, RNA, Peptide oder Effektormoleküle aller Art. Die Art der Bindung ist abhängig von Verwendungszweck des Tests, z.B. sollten alle potentiell Allergieauslösenden Moleküle kovalent gebunden sein, um eine ungewollte Immunisierung eines Patienten zu vermeiden.
Ferner können begleitend oder getrennt von den gebundenen Molekülen, freie lösliche Moleküle oder Substanzen oder Gemische von diesen, auf der Oberfläche der Vorrichtung appliziert sein. Diese dienen dazu biochemische Reaktionen auszulösen, z.B. Substanzen die eine Histaminausschütten von Mastzellen induzieren.

In der Vorrichtung können ziel gerichtet Analyten, Probenmaterial oder Zellen gesammelt werden. Diese zeil gerichtete Sammlung gescheit durch die gebundenen Moleküle oder Substanzen, an denen sich die Analyten oder Zellen binden und die Eigenschaften der Oberfläche der Vorrichtung an welches Probenmaterial bindet. Die Vorrichtung braucht nach einer endlichen Verweilzeit nur noch aus dem Rachenraum entfernt zu werden, vor der anschließend mit bekannten Verfahren die Messungen durchgeführt werden können.
Deshalb kann anschließend mit der erfindungsgemäßen Vorrichtung und Verfahren die Vielzahl der bestehenden analytischen Möglichkeiten wie Hochdruckflüssigkeitschromatograpfie (HPLC), Gaschromatographie (GC) aber auch Enzymimmunoassays (ELISA, RIA, EIA, RAST, EAST etc.) zur Messung der Körperantwort ohne große Änderungen genutzt werden. Es können aber auch Schnelltestverfahren mit der Vorrichtung gekoppelt werden, wie Lateral Flow -, Flow trough -, Solid phase - oder Agglutinations- Tests.
Die Vorrichtung wird an der Mundschleimhaut als Fixierungs- und Aktionsort im Körper des Patienten befestigt und kann, im Vergleich zu bestehenden Probennehmern oder Applikatoren, auf sehr milder und angenehmer Weise die ersten Schritte einer komplexen Aktion und Reaktion nachgebilden. Mit dem erfindungsgemäßen Vorrichtung können am lebenden Organismus (Mensch oder Tier) eine Vielzahl von Tests durchgeführt werden, ohne den Organismus des Patienten zu schädigen oder beeinträchtigen.

### Ziel der Erfindung

Ziel der in dieser Patentschrift vorgestellten Erfindung ist es, die bestehenden Nachteile der zur Zeit vorherrschenden Verfahren und Vorrichtungen für Probennahme und Applikation zu vermeiden. Ferner ein Testverfahren zur teilweisen Testsystem von Reaktionskaskaden aufzubauen, mit dem es möglich ist in vivo Reaktionen in vitro Messen zu können. Die Vorrichtung verbindet die nicht invasive Probennahme, eventuell vorgeschaltete Substanzapplikation, die Vorwegnahme einzelner biochemischer Kasakadenschritte und die universelle Einsetzbarkeit für nachfolgende Testverfahren.

Ein Nachteil der bestehenden Verfahren zur Probennahme ist es, das in vielen Fällen der Körper verletzt werden muss z.B. bei der Gewebeprobennahme. Dies ist mit Schmerzen und eventuell Blutungen (potentielle Infektionsgefahr für das Personal) verbunden. Bei nicht invasiven Probenentnahmeverfahren sind wiederum nicht alle analytischen Möglichkeit, die derzeit vorhanden sind nutzbar. Ferner werden bei der Probenentnahme viele biochemische Parameter verändert, z.B. durch Hormon, Mediator-Ausschüttung oder der Unterbrechung von Signalketten innerhalb von Gewebestrukturen.
Vergleichbar ist diese Situation auch bei den Applikatoren. Dort ist es das Problem, dass Substanzen oder Substanzgemische in den Körper gelangen und dort sowohl gewollte als auch ungewollte Reaktionen auslösen können. Dies ist besonders deutlich bei Medikamenten deren gewünschte Wirkung fast immer mit unerwünschten Nebenwirkungen begleitet ist oder bei Allergietest bei denen antigenes Material (hier Allergene) in den Körper gelangt, welches selbst wieder eine Allergie auslösen könnte. Beispiel:
Bei allergischen Erkrankungen möchte der behandelnde Arzt zuerst erfahren gegen welche Allergene der Patient eine allergische Reaktion zeigt. Werden jedoch, wie bei so genannten Provokationstests, die Allergene z.B. in die Nase appliziert, durch Einatmung oder durch Trägerfläche auf denen sich die Allergene befinden, können direkt oder durch Diffusion Allergene in den Körper gelangen. Damit soll die körpereigene Abwehrreaktion, im Falle eines positiven Allergens, bestimmt werden. Ungewollterweise werden möglicherweise aber erst eine Allergenisierung ausgelöst, dass heißt erst durch den Kontakt mit dem Allergen, das im Provokationstest eingesetzt wird, bildet sich eine neue Allergie gegen ebendieses Allergen aus. Ferner wird in der Therapie von Allergien die so genannte Desensibilisierung angewendet, bei der kleinsten Menge von Allergenen dem Patienten verabreicht werden. Z.B. der im Patent DE 19609315 vorgeschlagene nasale Probennehmer könnte eine ungewollte Sensibilisierung verursachen und damit dem Patienten Schaden zufügen.
Aufgabe der vorliegenden Erfindung war es diese Nachteile zu minimieren bzw. auszuschließen und dennoch ein für den Patienten erweitertes analytisch methodisches Spektrum zur Verfügung zu haben. Ferner war es die Aufgabe durch die Testsystem eines Teils des natürlichen Reaktionsweges, die gewünschten Testparameter in messbare Größen zu überführen zu können, ohne die Körperreaktionen im generellen beeinflussen zu müssen. Ferner sollte ein nicht invasives Verfahren geschaffen werden, dass den Patienten schont und schnell in der Durchführung ist.

### Beschreibung der Erfindung

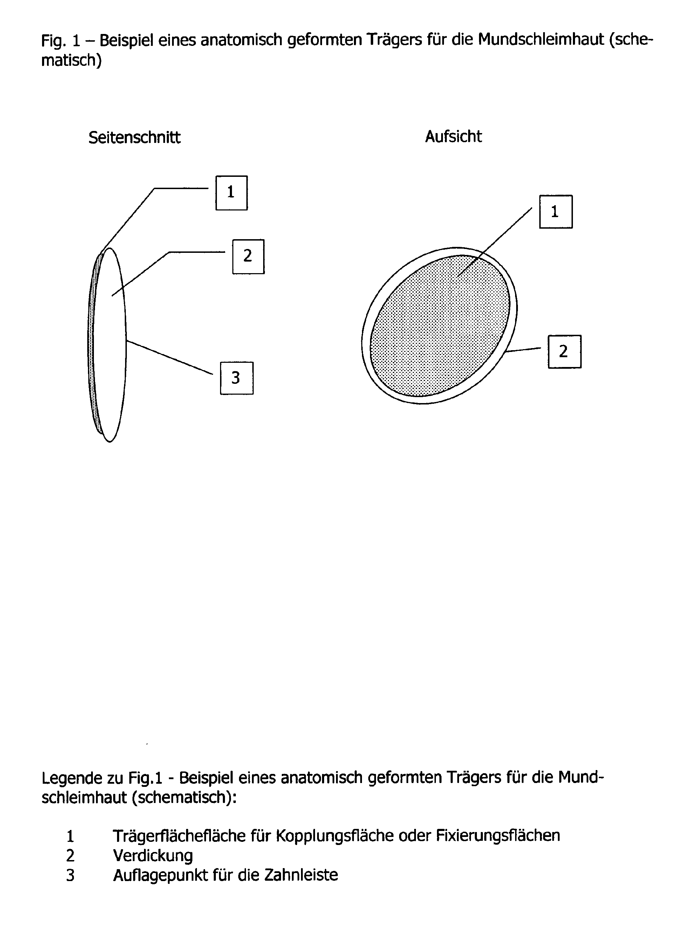

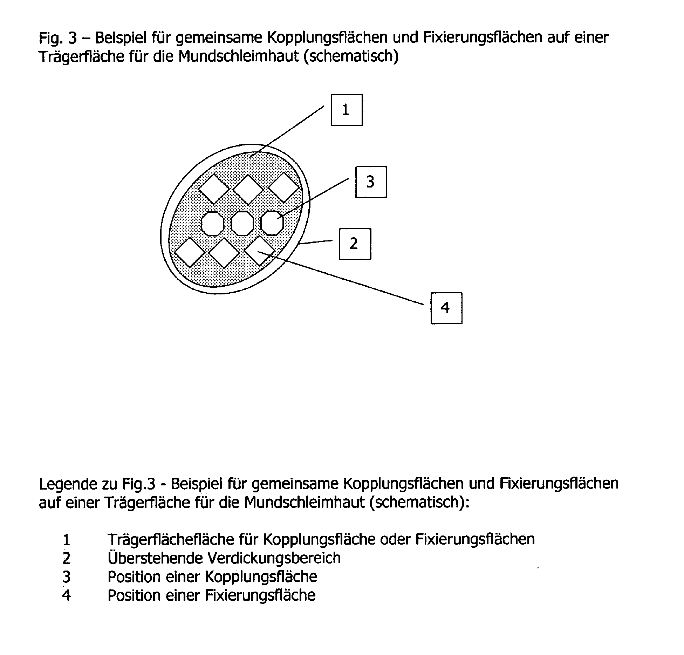

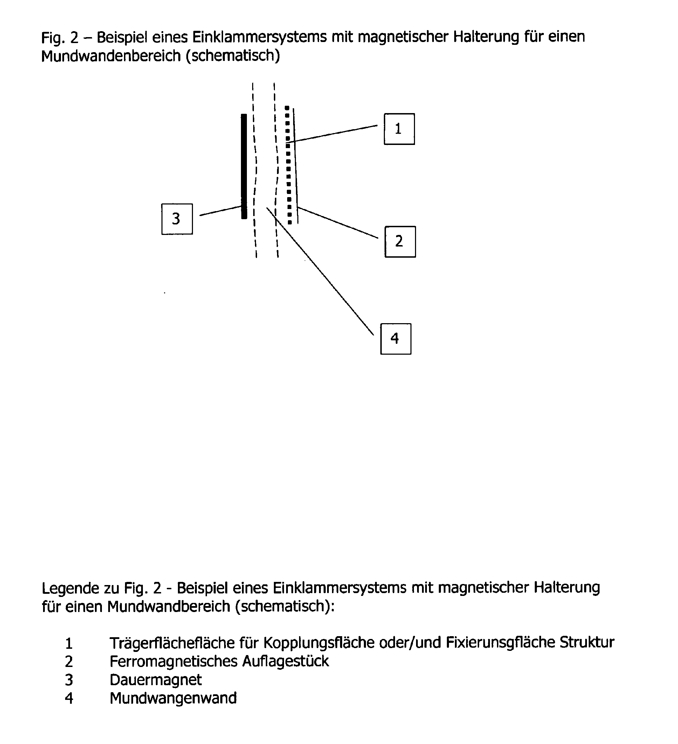

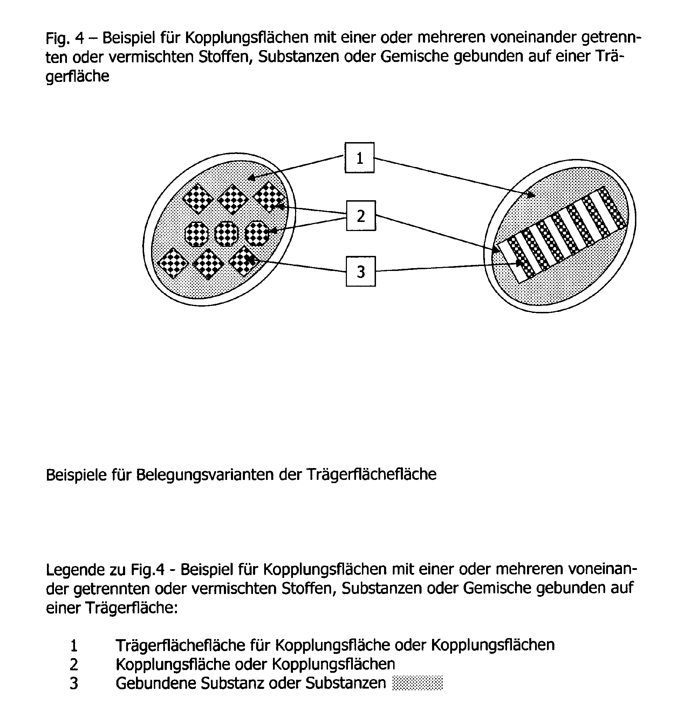

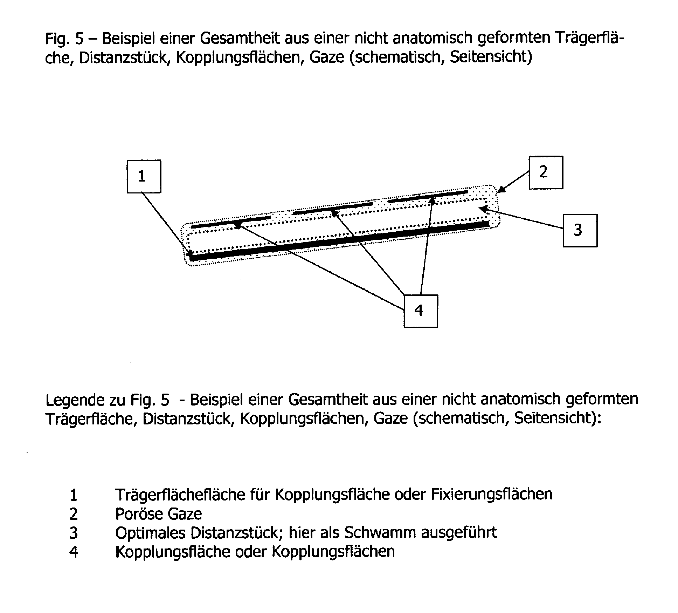

Die Aufgabe wird erfindungsgemäß durch eine Vorrichtung und Verfahren gelöst, dass es ermöglicht, mittels einer Trägerfläche eine oder mehrere Kopplungsflächen oder Strukturen auf der Mundschleimhaut für eine gewisse endliche Zeit zu fixieren.
Dabei sind Stoffe, Substanzen oder Gemische chemisch kovalent, chelatartig, mittel Van der Waals Kräfte oder ionisch auf der oder den Kopplungsfläche gebunden, so dass diese unter physiologischen Bedingungen nicht von der Kopplungsfläche entfernt werden oder diffundieren können. Eine oder mehrere Kopplungsflächen können sich direkt auf der Trägerfläche befinden, wobei die Moleküle oder Molekülgemische direkt auf der Trägeroberfläche gebunden sind. Ferner können die gebundenen Moleküle oder Molekülgemische über Bindungsschichten, wie Membranen, Folien, Platten oder Gewebe auf der Trägerfläche gebunden werden. Auf der oder den Kopplungsflächen, können die Moleküle oder Molekülgemische als Einzelpunkte und/oder als Arrays verschiedener Muster gebunden werden.
Die Kopplungsfläche oder -Flächen mit den gebundenen Stoffen, Substanzen oder Gemischen können mittels einer weit porigen Gase, welche aus verschiedenen Materialien ausgeführt werden kann, auf dem Trägerfläche befestigt oder ohne Gase durch geeignete Verfahren befestigt, z.B. durch verkleben, verschweißen, klemmen, nageln, nieten etc. werden.
Der Trägerfläche selbst ist als feste aber flexible Unterlage ausgebildet, wobei sich eine leicht gewölbte ovale Form, welche dem Rachenraum an der Mundschleimhaut angepasst ist (Fig.1) sich als besonders tauglich erwiesen hat. Die Größen können variieren und müssen dem physiologischen Verhältnissen angepasst werden, z.B. eine kleine Trägerfläche für Kinder und größere Flächen für Erwachsene. Die Trägefläche ist derart gestaltet, das bei geschlossen Mund, diese durch die Zahnleiste an der Mundschleimhaut gehalten im Wangenbereich wird. (Fig.1). Ferner kann die Trägerfläche aus verschiedenen Materialien aufgebaut werden, z.B. Kunststoffe, natürliche Polymere wie Cellulose, Keramik, Glas, Metall etc., welche den Patentumfang nicht einschränken. Ferner die Trägerfläche aus Komposit-Materialien aufgebaut sein. Besonderes vorteilhaft haben sich Kunststoffe mit magnetischen Partikeln erwiesen. Damit kann die Trägerfläche durch, von außen angelegte Magneten, an die innere Schleimhaut gepresst werden (Fig. 2). Über eine Abstimmung der magnetischen Haltekräfte kann der oder die Trägerflächen, samt den gebundenen Kopplungsflächen, mit optimalen Druck an die Mundschleimhaut gepresst werden.
Neben den Kopplungsflächen kann die Trägerfläche auch lösliche Moleküle oder Molekülgemische enthalten, welche als freie Schicht oder mittels Membran, Gase, Gewebe fixiert ist, solange diese sich nicht in der Mundschleimhaut befindet. (Fig. 3). Gebundene, wie freie Moleküle oder Molekülgemische können sich gemeinsam oder einzeln auf der Trägerfläche befinden (Fig. 4).
Im Gegensatz zu Medikamenten welche in die Schleimhäute diffundieren sollen, werden hier Moleküle oder Molekülgemische verwendet, welche eine Reaktion auslösen können und damit einen physiologischen Zustand analysierbar und messbar machen. Es handelt sich dabei nicht um die Gabe eines Medikamentes. Beispiel, welches den Patentumfang nicht einschränkt:
Wird Inter-Leukin-4 als freies Molekül über die Trägerfläche auf die Schleimhaut gebracht, so reagieren die Helfer-T-Lymphozyten u.a. mit der Produktion von IgE-Antikörpern. Diese IgE-Antikörper können mittels auf der Trägerfläche gebundener Anti-IgE-Antikörper quantifiziert werden und auf ihre Spezifität Untersucht werden. die Konzentration der freigesetzten IgE-Antikörper ist das Maß für die Ausprägung einer Allergie. Die Spezifität der freigesetzten IgE-Antikörper liefert die Aussage, gegen welches Antigen (in diesem Falle Allergen) der Patienten eine Allergie ausgebildet hat.
Die Kopplungsflächen oder Strukturen auf der die Stoffe, Substanzen oder Gemische gebunden sind kann mehrfach geteilt oder aus einem Stück gefertigt sein. Auf der Kopplungsfläche oder Struktur kann eine oder mehrere von einander getrennte oder vermischte Stoffe, Substanzen oder Gemische gebunden sein oder es können mehrere voneinander getrennte Kopplungsflächen oder Strukturen auf dem Trägerfläche befestigt sein (Fig.4).
Ferner kann auf der Trägerfläche unter der Kopplungsfläche oder Struktur ein Distanzstück (Fig.5) in Form eines Schwammes oder Gewebes gleich welcher Struktur oder Härte aufgebracht werden. Trägerfläche, Distanzstück, Kopplungsfläche und Gase können in verschiedenen Materialien, Formen ausgeführt werden. Ohne die Patentansprüche einzuschränken kann als Beispiel, die Ausgestaltung der Form in anatomisch der Mundschleimhaut oder der Darmmucosa angepasster Art ausgeführt werden.
Die Gesamtheit aus Trägerfläche mit oder ohne Distanzstück mit Kopplungsfläche oder Struktur mit oder ohne poröser Gase mit oder ohne löslichen Molekülen oder Molekülgemischen als Schicht oder fixiert mittels Gase, Gewebe etc. stellt die erfindungsgemäße Vorrichtung dar.
Diese Vorrichtung ist die Basis für das erfindungsgemäße Verfahren des Systems der Testung eines Analyten und für die folgenden analytischen Schritte. Das erfindungsgemäße Verfahren verwendet kovalent oder chelatartig gebundene Stoffe, Substanzen oder Gemische, diese werden an die Mundschleimhaut gebracht indem die Trägerfläche oder -Flächen samt der Kopplungsfläche oder -Flächen und/oder Fixierungsfläche oder -Flächen, an die Mundschleimhaut presst. Damit Kann die wird die Exposition der Mundschleimhaut mit einem Stoff simuliert werden oder auf aktiven Weg Analyte, z.B. durch Antigen/Antikörperreaktionen oder DNA/RNA Sonden gebunden werden. Durch die kovalent oder chelatartige Bindung wird verhindert, dass die auf der Kopplungsfläche oder Struktur gebundenen Stoffe, Substanzen oder Gemische in die Mundschleimhaut und damit in den Körper des Patienten eindringen können und durch einfache Entnahme der erfindungsgemäßen Trägerfläche in nachgeschalteten Analyseverfahren untersucht werden. Im Gegensatz zur passiven Entnahme von Proben, wie Gewebe oder Schleimhautflüssigkeit, stellt das erfindungsgemäße Verfahren eine nicht invasive und zielgerichtetes Analytischen Verfahren dar, welches die Möglichkeiten einer späteren (in vitro) Analytik nicht einschränkt. Ferner können durch die gezielte und simultane Stimulation mittels Effektormoleküle in vivo Tests durchgeführt werden, was durch eine Probennahme nicht möglich ist.
Als ein Beispiel, welches den Patentumfang nicht einschränkt, sind die allergischen Erkrankungen zu nennen. Die häufigste Erkrankung ist die Rhinitis allergica, deren Morbidität in Mitteleuropa mit 10 bis 20% in der wissenschaftlichen Literatur angegeben wird. Natürlicherweise trifft ein in der Atemluft befindliches Allergen auf die Mundschleimhaut. Die dort vorhandenen Mastzellen samt IgE Antikörpern und weiteren Immunglobuline erkennen das oder die Allergene. Im positiven Fall wird eine Reaktionskaskade ausgelöst, welche anschließend die Erkrankungs üblichen Symptome der zur Folge hat.
Der erfindungsgemäße Vorrichtung, hat ein oder mehrere Allergene auf der Kopplungsfläche welche Kontakt zur Mundschleimhaut hat, gebunden. Es wird also die Exposition der Mundschleimhaut mit einem Allergen und die Bindung des Antikörper bzw. einer Zelle an das Allergen simuliert und damit den der Hauptteil des Beginns einer allergischen Kaskade, die zur Auslösung der Symptome einer allergischen Erkrankung führt.
Die IgE Antikörper oder andere Subklassen der Immunglobuline binden im positiven Falle an die gebundenen Allergene auf der Kopplungsfläche. Da die Antikörper nach der Bindung an das Allergen ebenfalls gebunden sind, können sie nicht mehr das Signal zum Start der Reaktionskaskade auslösen; der Patient bleibt Beschwerde frei und kann nicht immunisiert (allergenisiert) werden. Nach Entfernen der Trägerfläche von der Mundschleimhaut, samt Kopplungsfläche, kann die Konzentration der gebundenen IgE Antikörper oder anderer Immunsubklassen außerhalb des Körper mit dem Stand der Technik entsprechenden in vitro ELISA Methoden gemessen werden. Die gemessene Konzentration an allergenspezifischen IgE-Antikörper ist ein international akzeptiertes Maß für die Ausprägung einer allergischen Erkrankung vom Typ-1. Da auch Zellen und andere Immunglobulin Subklassen an die Allergene auf der Kopplungsfläche im positiven Fall binden, können auch andere Typen von allergischen Erkrankungen nachgewiesen werden.
Als ein weiteres Beispiel, welches ebenfalls den Patentumfang nicht einschränkt, ist die Verlaufskontrolle von Medikamentengaben bei Patienten. In diesem Fall sind Antikörper gegen die Wirksubstanz des Medikamentes oder und deren Metaboliten an der Kopplungsfläche oder Struktur kovalent gebunden. Wird die Kopplungsfläche in definierten Abständen mittels der erfindungsgemäßen Trägerflächen an die Mundschleimhaut gebracht, binden die spezifischen Antikörper das Medikament oder und deren Metabolite. Nach entfernen der Kopplungsfläche samt der gebundenen Medikamentmoleküle oder und deren Metabolite können pharmakologische Daten wie Blut- oder Gewebekonzentration zeitlich aufgelöst bestimmt werden, in dem klassische *in vitro* analytische Methoden wie z.B. HPLC oder GC angewendet werden. Damit kann das Anfluten von Medikamenten oder deren Metabolite im Speichergewebe des Körpers wie Fettgewebe oder Muskelgewebe zeitlich aufgelöst simuliert werden.
Das erfindungsgemäße Verfahren stellt die Gesamtheit aus kovalenter oder chelatartiger Bindung eines oder mehrer Stoffe, Substanzen oder Gemische, deren Exposition mittels der erfindungsgemäßen Trägerfläche an der Mundschleimhaut und die Verbindung mit einem nach dem Stand der Technik vorhandenen analytischen Verfahren dar.
Als ein weiteres Beispiel, welches ebenfalls den Patentumfang nicht einschränkt, ist die Bestimmung von zirkulierenden Antikörpern, wie Autoimmunantikörpern wie, ANA-Antikörper, PA-IgG Antikörper oder Antikörpern die in der Infektionsserologie eine Rolle spielen, wie Hepatitis A, B Antikörper, HIV-Antikörpern etc.
Als ein weiteres Beispiel, welches ebenfalls den Patentumfang nicht einschränkt, ist die Bestimmung von freier DNA oder RNA mittel gebundener Fangsonden. Diese freien Polynukleinsäuren können zur Bestimmung von Viren, Bakterien, Pilzen oder anderen Organismen, welche sich im Organismus befinden oder vorhanden waren eingesetzt werden.

### Anhänge

### Verzeichnis der Zeichnungen

1 Fig. 1 - Beispiel eines anatomisch geformten Trägers für die Mundschleimhaut (schematisch)
2 Fig. 2 - Beispiel eines Einklammersystems mit magnetischer Halterung für einen Mundwandenbereich (schematisch)
3 Fig. 3 - Beispiel für gemeinsame Kopplungsflächen und Fixierungsflächen auf einer Trägerfläche für die Mundschleimhaut (schematisch)
4 Fig. 4 - Beispiel für Kopplungsflächen mit einer oder mehreren voneinander getrennten oder vermischten Stoffen, Substanzen oder Gemische gebunden auf einer Trägerfläche
5 Fig. 5 - Beispiel einer Gesamtheit aus einer nicht anatomisch geformten Trägerfläche, Distanzstück, Kopplungsflächen, Gaze (schematisch, Seitensicht)

### Verzeichnis der Literatur

1 US 4,800,896 vom 31.01.1989
2 US 4,887,611 vom 19.12.1989
3 F. Marcucci und L. Sensi, A new method for IgE detection in nasal mucosa, Clinical and Experimental Allergy, 1989 Vol.19, Seite 157 - 162
4 DE 19609315
5 DE69112610T2 (Sammlung von Analyten aus der Mundschleimhaut für Immunoassays)
6 DE69423342T2 (Transmukosale Verabreichung von makromolekularen Medikamenten)
7 DE3618553C2 (Medizinischer Klebestreifen für die Mundschleimhaut)
8 DE19622503C2 (Verfahren zum Nachweis von Analyten auf einer Oberfläche)

## Patentansprüche

1. Vorrichtung und Verfahren als Vorrichtung benannt **dadurch gekennzeichnet, dass** eine Vorrichtung verwendet wird, die mittels einer Trägerfläche gleich welchen Materials oder Form eine oder mehrere Kopplungsflächen gleich welchen Materials, Form, Dicke an die Mundschleimhaut für einen endlichen Zeitraum in vivo gepresst, auf der kovalent eine oder mehrere Moleküle oder Molekülgemische gebunden sind und nachdem zeitlich begrenzten Zeitraum von der Mundschleimhaut entfernt werden kann, sodass anschließend die Kopplungsfläche vom Trägerfläche entfernt werden kann und mit dieser oder diesen, außerhalb der Mundschleimhaut in vitro analytische Untersuchungen durchgeführt werden können, welche als Hochdruckflüssigkeitschromatograpfie, Gaschromatographie, Elektrophorese Analysetechniken oder Spektroskopischen Verfahren, aber auch Enzymimmunoassays (ELISA, RIA, EIA, RAST, EAST etc.), Schnelltestverfahren mit der Vorrichtung gekoppelt werden, wie Lateral Flow -, Flow trough -, Solid phase - oder Agglutinations- Tests ausgeführt werden. Ferner das zwischen Kopplungsfläche und Trägerfläche ein Anstandshalter welcher in verschiedene Materialien, Härten, Dicken und Porositäten ausgeführt werden, zwischen gelegt werden kann.

2. Vorrichtung nach Anspruch 1, **dadurch gekennzeichnet**, das diese den anatomischen Verhältnissen der Mundschleimhaut oder der Darmmucosa angepasst ist.

3. Trägerfläche nach Anspruch 1 bis 2, **dadurch gekennzeichnet, dass** dieser zusätzlich eine oder mehrere Bereiche als Fixierungsbereiche enthält, welche frei lösliche Moleküle oder Molekülgemische aufnehmen können, ausgestaltet als Schicht, Gewebe, Filter, Gase oder das diese direkt auf die Trägervorrichtung Vorrichtung gemäß Anspruch 1 aufgebracht ist.

4. Trägerfläche nach Anspruch 1 bis 3, **dadurch gekennzeichnet, dass** dieser als Klammer ausgeführt ist, welche einen Trägerfläche an die innen Seite an je einer äußeren Wangenwand fixieren kann.

5. Trägerfläche nach Anspruch 1 bis 3, **dadurch gekennzeichnet, dass** dieser aus zwei getrennten Teilen aufgebaut ist, wobei ein Teil der Trägerfläche entspricht welcher an die innen Seite an einer äußeren Mundwand mittels magnetischer Kraft fixiert werden kann und die andere Teil den dazu notwendigen Magneten, ausgeführt als Dauermagnet oder Elektromagnet, aufnimmt.

6. Kopplungsfläche nach Anspruch 1, **dadurch gekennzeichnet, dass** diese aus verschiedenen Materialien, wie Kunststoffe, natürliche Kunststoffe, Glas, Keramik, Metall und Gemischen aus diesen, ausgeführt ist und diese Materialien in verschiedenen Formen, Dicken, Netzen oder Poren ausgeführt werden können. Ferner **dadurch gekennzeichnet, dass** eine oder mehrere Moleküle oder Molekülgemische einzeln oder von einander getrennt chemisch kovalent, mittels Chelatkomplexierung, ionisch Wechselwirkung oder per Van der Waals Kräfte gebunden werden können.

7. Fixierungsfläche nach Anspruch 3, **dadurch gekennzeichnet, dass** diese aus verschiedenen Materialien, wie Kunststoffe, natürliche Kunststoffe, Glas, Keramik, Metall und Gemischen aus diesen, ausgeführt ist und diese Materialien in verschiedenen Formen, Dicken, Netzen oder Poren ausgeführt werden können. Ferner, **dadurch gekennzeichnet, dass** diese durch eine netzartige poröse Gaze, welche aus verschieden Materialien bestehen kann fixiert wird oder das die Fixierung durch Verklebung, klemmen, nageln oder nieten ausgeführt wird.

8. Abstandshalter nach Anspruch 1 bis 2 **dadurch gekennzeichnet, dass** dieser als Schwamm oder anderes weiches Material ausgeführt wird.

9. Verfahren mit der Vorrichtung nach Anspruch 1 bis 7, **dadurch gekennzeichnet, dass** eine oder mehrere Kopplungsflächen nach Anspruch 6 mit einem oder mehreren Allergenen oder -gemischen aus diesen kovalent beschichtet sind und zum Nachweis von allergischen Erkrankungen, Infektions-, Autoimmun oder Stoffwechselerkrankungen genutzt werden. Ferner **dadurch gekennzeichnet, dass** eine oder mehrere Kopplungsflächen nach Anspruch 6 mit einem oder mehreren Antikörpern oder Gemischen aus diesen kovalent beschichtet sind und zum Nachweis von Substanzen in der Mundschleimhaut genutzt werden kann. Ferner das diese auch mit einer zeitlich festgelegten Reihenfolge an die Mundschleimhaut eingesetzt werden. Ferner **dadurch gekennzeichnet, dass** eine oder mehrere Kopplungsflächen nach Anspruch 6 mit einem oder mehreren Substanzen oder Gemischen aus diesen kovalent beschichtet sind und zum Nachweis von Mediatoren, Antikörpern, Antigenen, Enzymen und Enzymaktivitäten, Peptiden, RNA, DNA oder anderen bindungsfähigen Substanzen in der Mundschleimhaut genutzt werden kann. Ferner das diese auch mit einer zeitlich festgelegten Reihenfolge an die Mundschleimhaut eingesetzt werden.

10. Verfahren mit der Vorrichtung nach Anspruch 1 bis 7, **dadurch gekennzeichnet, dass** eine oder mehrere Fixierungsflächen nach Anspruch 7 mit einem oder mehreren Allergenen oder -gemischen belegt sind und zum Nachweis von allergischen Erkrankungen, Infektions-, Autoimmun oder Stoffwechselerkrankungen genutzt werden. Ferner **dadurch gekennzeichnet, dass** eine oder mehrere Kopplungsflächen nach Anspruch 7 mit einem oder mehreren Antikörpern oder Gemischen belegt sind und zum Nachweis von Substanzen in der Mundschleimhaut genutzt werden kann. Ferner das diese auch mit einer zeitlich festgelegten Reihenfolge an die Mundschleimhaut eingesetzt werden. Ferner **dadurch gekennzeichnet, dass** eine oder mehrere Fixierungsflächen nach Anspruch 7 mit einem oder mehreren Substanzen oder Gemischen belegt sind und zum Nachweis von Mediatoren, Antikörpern, Antigenen, Enzymen und Enzymaktivitäten, Peptiden, RNA, DNA oder anderen bindungsfähigen Substanzen in der Mundschleimhaut genutzt werden kann.
